# EUROPEAN PATENT APPLICATION

(11) **EP 2 383 248 A1**
(43) Date of publication of application: **02.11.2011**
(21) Application number: 10004545.9
(22) Date of filing: 29.04.2010
(51) Int. Cl.: C07C 45/69, C07C 49/227, C07C 49/80

(54) **Process for the preparation of beta-chlorovinyl ketones**

(71) Applicant: Lonza Ltd, 4052 Basel (CH)
(72) Inventor: Dressel, Martina, 3902 Brig-Glis (CH); Ott, Lothar, 3930 Visp (CH); Snelders, Dennis, 1006 Lausanne (CH); Dyson, Paul, 1024 Ecublens (CH)

(57) **Abstract**

β-Chlorovinyl ketones are prepared by reacting acyl chlorides with acetylene in chloroaluminate-containing ionic liquids. The process gives high yields without formation of tarry byproducts.

## Description

The invention relates to a process for the production of β-chlorovinyl ketones of formula

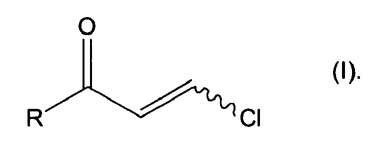

β-Chloro-α,β-unsaturated ketones are used as synthetic intermediates in a wide variety of aromatic, aliphatic and heterocyclic compounds, many of which are important to the chemical and pharmaceutical industries. A range of compounds of this type are accessible via an addition reaction of alkynes with acyl chlorides in the presence of a stoichiometric amount of AlCl₃. β-Chlorovinyl ketones, a special class of highly versatile intermediates, have been prepared by reacting acyl chlorides with acetylene. This method works for a wide range of substrates, with yields of typically 50-70%, but usually requires the use of chlorinated solvents such as dichloroethane or carbon tetrachloride, which are highly toxic and damaging to the environment. Moreover, it has been found that in chlorinated solvents the reaction is often accompanied by the formation of considerable amounts of tar, apparently by aluminum chloride-catalyzed polymerization of acetylene. Transition metal-catalyzed methods for the addition of acyl chlorides to terminal alkynes are known, but these methods require expensive rhodium- or iridium-based catalysts and the use of acetylene as substrate has not been reported. It is therefore an object of the present invention to provide a new synthetic method for the synthesis of β-chlorovinyl ketones that combines the use of cheap and environmentally less hazardous reagents with high product yields and low tar formation.

According to the invention, this object has been achieved by the process of claim 1.

It has been found that β-chlorovinyl ketones of formula 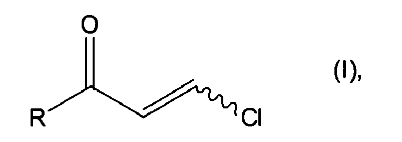 wherein R is selected from the group consisting of linear and branched C₁₋₂₀ alkyl, C₂₋₂₀ alkenyl, C₃₋₁₀ cycloalkyl, alkyl-substituted C₃₋₁₀ cycloalkyl, aryl, substituted aryl, heteroaryl, substituted heteroaryl, arylalkyl, substituted arylalkyl, arylalkenyl, substituted arylalkenyl, heteroarylalkyl, and substituted heteroarylalkyl, can be prepared in good yields by reacting an acyl chloride of formula 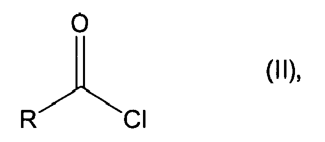 wherein R is as defined above,
with acetylene in a ionic liquid comprising a chloroaluminate of formula

Q⁺ [AlₙCl₃ₙ₊₁]⁻ (III),

wherein *n* is 1 or 2, and Q⁺ is an organic cation selected from the group consisting of 1,3-dialkylimidazolium, wherein both alkyl groups are independently C₁₋₁₀ alkyl; and optionally alkyl-substituted *N*(C₁₋₁₀ alkyl)pyridinium.

Herein, "C_{1-*n*} alkyl" means any linear or branched alkyl group having from 1 to *n* carbon atoms. For example, "C₁₋₂₀ alkyl" comprises groups such as methyl, ethyl, propyl, isopropyl, butyl, isobutyl, *sec-*butyl, *tert-*butyl, pentyl, isopentyl, *tert-*pentyl, neopentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, and any isomers of the foregoing.

Accordingly, "C₂₋ₙ alkenyl" means any linear or branched hydrocarbyl group having from 2 to *n* carbon atoms and at least one double bond in any position. For example, "C₂₋₂₀ alkenyl" comprises groups such as vinyl, 1-propenyl, allyl (2-propenyl), isopropenyl, methallyl (2-methylallyl), 1-butenyl, 2-butenyl, 3-butenyl or oleyl (octadec-9-enyl).

"C₃₋₁₀ cycloalkyl" comprises any mono- or bicyclic saturated hydrocarbyl group having 3 to 10 carbon atoms, such as cyclopropyl, cyclobutyl, cyclopentyl, cyclohexyl, cycloheptyl, cyclooctyl, cyclononyl, cyclodecyl, norbornyl, norcaranyl, or norpinanyl. These cycloalkyl groups may be substituted with one or more halogen atoms or C₁₋₄ alkyl groups, in particular methyl or isopropyl groups. Exemplary substituted cycloalkyl groups are bornanyl, caranyl, methanyl, pinanyl, thujanyl, including their isomers and stereoisomers.

"Aryl" comprises any mono-, bi- or polycyclic hydrocarbyl group that contains at least one aromatic ring, such as phenyl, naphthyl, biphenylyl, anthracenyl, phenanthrenyl, terphenylyl, pyrenyl, and the like. A bi- or polycyclic aryl group may also contain one or more non-aromatic rings, like fluorenyl or tetrahydronaphthalenyl, provided that it is attached via one of its aromatic rings.

Any of the foregoing aryl groups may be substituted with one or more halogen atoms, in particular fluorine, chlorine or bromine, or with one or more nitro or C₁₋₄ alkyl groups, or with any combination of any of the foregoing.

"Heteroaryl" comprises any mono-, bi- or polycyclic moiety that is attached via one of its carbon atoms and that contains at least one five- or six-membered heteroaromatic ring containing one or more heteroatoms, independently selected from the group consisting of nitrogen, oxygen and sulfur. In addition to the heteroaromatic ring(s), a heteroaryl moiety may contain one or more aromatic or non-aromatic ring(s). Examples of heteroaryl groups are pyrrolyl, pyrazolyl, imidazolyl, pyridinyl, pyrimidinyl, pyrazinyl, pyridazinyl, triazinyl, furanyl, oxazolyl, isoxazolyl, thiazolyl, isothiazolyl, indolyl, quinolinyl, carbazolyl, phenazinyl, and the like.

Any heteroaryl moiety may be substituted in the same way as indicated above for aryl.

"Arylalkyl" means an alkyl group, preferably a C₁₋₄ alkyl group, that is substituted with one or more aryl groups as defined above. Examples of arylalkyl groups are benzyl, diphenylmethyl (benzhydryl), triphenylmethyl (trityl), 1-phenylethyl, 2-phenylethyl (phenethyl), 3-phenylpropyl (hydrocinnamyl), naphthylmethyl, and the like.

The aryl part of any arylalkyl moiety may be substituted in the same way as indicated above for aryl.

"Arylalkenyl" means an alkenyl group, preferably a C₂₋₄ alkenyl group, that is substituted with one or more aryl groups as defined above. Examples of arylalkenyl groups are 2-phenylvinyl (styryl) and 3-phenylallyl (cinnamyl).

The aryl part of any arylalkenyl moiety may be substituted in the same way as indicated above for aryl.

"Heteroarylalkyl" means an alkyl group, preferably a C₁₋₄ alkyl group, that is substituted with one or more heteroaryl groups as defined above. Examples of heteroarylalkyl groups are pyridylmethyl, thiophenylmethyl (thenyl) and quinolin-2-ylmethyl (quinaldyl).

The aryl part of any heteroarylalkyl moiety may be substituted in the same way as indicated above for aryl.

The cation Q⁺ is selected from 1,3-di(C₁₋₁₀ alkyl)imidazolium and optionally alkyl-substituted *N*-(C₁₋₁₀ alkyl)pyridinium.

In 1,3-di(C₁₋₁₀ alkyl)imidazolium, the two alkyl groups may be linear or branched and may have the same or different numbers of carbon atoms, but are preferably linear with different chain lengths, such as methyl and butyl, methyl and pentyl, ethyl and propyl, ethyl and butyl, and similar combinations.

In optionally alkyl-substituted *N*-(C₁₋₁₀ alkyl)pyridinium, the alkyl group attached to the nitrogen atom is preferably a linear C₂₋₆ alkyl group, more preferably an n-butyl group. The optional alkyl-substitution may comprise one or more C₁₋₁₀ alkyl groups attached to the ring carbon atoms of the pyridine nucleus. Preferred alkyl groups are methyl and ethyl.

In a preferred embodiment, the cation Q⁺ is 1-butyl-3-methylimidazolium or Mbutyl-3-methylpyridinium.

In another preferred embodiment, R is selected from the group consisting of linear and branched C₁₋₂₀ alkyl, phenyl, and substituted phenyl. In substituted phenyl the substituent(s) may be those listed above in the definition or substituted aryl.

The chloroaluminate (III) in the ionic liquid is advantageously prepared from the corresponding chloride of formula

Q⁺ Cl⁻ (IV),

wherein Q⁺ is as defined above, and anhydrous aluminum trichloride.

The formation of the chloroaluminate can be described by the following equations:

Q⁺ Cl⁻ + AlCl₃ ⇄ Q⁺ [AlCl₄]⁻ (III, *n* = 1)

Q⁺[AlCl₄]⁻ + AlCl₃ ⇄ Q⁺[Al₂Cl₇]⁻ (III, *n* = 2)

The relative amounts of the [AlCl₄]⁻ and [Al₂Cl₇]⁻ species formed depend on the molar ratio of aluminum trichloride and the chloride (IV).

In a preferred embodiment, the molar ratio of aluminum trichloride and the chloride (IV) is in the range from 1.5 to 3.0, more preferably from 1.8 to 2.5, and most preferably from 2.0 to 1.3, corresponding to a mole fraction of AlCl₃ in the ionic liquid of 0.66-0.70.

The chloroaluminate-containing ionic liquid is preferably used in an amount that results in a ratio of the difference of the molar amounts of aluminum trichloride and the chloride (IV) to the molar amount of acyl chloride (II) in the range from 1.0 to 2.5, more preferably from 1.15 to 2.0, and most preferably from 1.3 to 1.8.

The process of the invention is advantageously carried out in two steps, by first adding the acyl chloride (II) to the ionic liquid comprising the chloroaluminate (III), and then adding acetylene to the resulting mixture.

It has been found that, in contrast to the conditions required when using chlorinated solvents, the reaction temperature in the first step is not critical, in particular it is not necessary to cool the reaction mixture below room temperature.

The appropriate reaction temperature in the second step, the addition of acetylene to the mixture of acyl chloride (II) and chloroaluminate-containing ionic liquid, depends on the reactivity of the acyl chloride. For example, it has been found that with aroyl chlorides such as benzoyl chloride (II, R = Ph) or substituted benzoyl chlorides as starting materials the second step is advantageously carried out at temperatures of 40-80 °C, more preferably 50-60 °C, while alkanoyl chlorides are preferably reacted at temperatures of -10 to +20 °C, more preferably at about 0 °C.

The β-chlorovinyl ketones (I) can exist in two isomeric forms *(cis*/*trans* isomerism, *Z* and *E* isomers), the *E* isomer of which is preferred. It has been found that at least in some cases there is an equilibrium between both forms that is reached only after several hours.

The following non-limiting examples are intended to illustrate the invention in detail.

### Example 1 (Comparative Example)

### Preparation of β-chlorovinyl phenyl ketone (I, R = Ph) in 1,2-dichloroethane (typical procedure)

A suspension of anhydrous aluminum trichloride (0.728 g, 5.46 mmol) in 1,2-dichloroethane (DCE; 2.5 mL) was cooled to 0 °C (*T*₁). Benzoyl chloride (II, R = Ph; 0.51 mL, 4.39 mmol) was added dropwise, upon which the reaction mixture turned clear. The mixture was stirred at 0 °C for 15 min. The mixture was then heated to 50 °C (*T*₂) and acetylene was bubbled through the mixture via a syringe during 2 h, upon which the mixture turned black and viscous. The mixture was then poured into water (50 mL) and extracted with CH₂Cl₂ (3×50 mL). The organic layer was dried with anhydrous MgSO₄, filtered and concentrated. The resulting black oil was purified by column chromatography using silica gel 60 M and EtOAc/hexane (1/9 (v/v)) as eluent. The product (*R_{f}* = 0.42) was obtained as a yellow liquid.
Yield: 0.44 g (60%).
¹H NMR (CDCl₃, 400 MHz): δ 7.93 (d, 2H, *J*= 7.4 Hz), 7.60 (t, 1H, *J*= 7.4 Hz), 7.50 (t, 2H, *J*= 7.6 Hz), 7.47 (d, 1H, *J*= 13.1 Hz), 7.32 (d, 1H, *J*= 13.2 Hz).

### Example 2

### Preparation of β-chlorovinyl phenyl ketone (I, R = Ph) in an ionic liquid (typical procedure)

Anhydrous aluminum trichloride (1.83 g, 13.8 mmol) and *N*-butyl-3-methylpyridinium chloride ([bmpy]⁺Cl⁻, prepared according to WO 2005/097749 A1; 1.09 g, 5.88 mmol) were placed in a Schlenk tube under a nitrogen atmosphere and stirred at room temperature (-25 °C, *T*₁) for 30 min until a clear, homogeneous liquid had formed. Benzoyl chloride (II, R = Ph; 0.50 mL, 4.30 mmol) was added dropwise at room temperature, upon which the reaction mixture became a clear dark yellow liquid. The mixture was then stirred at room temperature for 15 min, during which the mixture became a thick suspension. The mixture was then heated to 60 °C (*T*₂), rendering the mixture a clear solution again, and acetylene was bubbled through the mixture during 2 h, upon which the mixture slowly turned dark brown. The mixture was then poured into ice water (50 mL) and the dark color disappeared. The product was extracted with petroleum ether (3×50 mL). The organic layer was washed with water (3×50 mL), dried with anhydrous MgSO₄, filtered and concentrated. The product was obtained as a yellow-orange liquid.
Yield: 0.66 g (93%).

### Example 3

The procedures of Examples 1 and 2 were repeated using different molar ratios of aluminum trichloride and acyl chloride (II), different amounts of chloroaluminate in the ionic liquid (IL), if applicable, and at different temperatures. 1-Butyl-3-methylimidazolium chloride ([bmim]⁺Cl⁻) was purchased from Sigma-Aldrich. The results are compiled in Table 1 below:

**Table 1**

| **Entry** | **Medium** | **Mole Fraction of AlCl₃ in IL** | **Molar Ratio AlCl₃/PhCOCl** | ***T*₁ (°C)** | ***T*₂ (°C)** | **Conversion (@ 1 h, %)** | **Conversion (@ 2 h,%)** | **Tar** | **Isolated Yield (%)** | **Purity (GC, %)** | ***E*/ZRatio (GC)** |
|---|---|---|---|---|---|---|---|---|---|---|---|
| 1 | DCE | - | 1.04 | 25 | 50 | 51 | n.d. | no | n.d. | n.d. | n.d. |
| 2 | " | - | 1.25 | 25 | 50 | 80 | " | some | " | " | " |
| 3 | " | - | 1.04 | 0 | 50 | 65 | 79 | no | " | " | " |
| 4 | " | - | 1.25 | 0 | 50 | 93 | 98 | yes | 60 | 98 | 98/2 |
| 5 | [bmim]⁺[Al*ₙ*Cl_{3*n*+1}]⁻ | 0.66 | 1.05 | 0 | 50 | 85 | 87 | no | n.d. | n.d. | n.d. |
| 6 | " | 0.66 | 1.25 | 0 | 50 | 91 | 93 | " | " | " | " |
| 7 | " | 0.66 | 1.25 | 25 | 50 | 89 | 95 | " | " | " | " |
| 8 | " | 0.67 | 1.25 | 25 | 60 | 94 | 99 | " | 71 | 98 | 98/2 |
| 9 | " | 0.62 | 0.79 | 0 | 60 | 41 | 47 | " | n.d. | n.d. | n.d. |
| 10 | [bmpy]⁺[Al*ₙ*Cl_{3*n*+1}]⁻ | 0.67 | 1.25 | 25 | 50 | n.d. | 96 | " | " | " | " |
| 11 | " | 0.67 | 1.25 | 25 | 60 | 97 | >99 | " | 74 | 98 | 98/2 |

"Mole Fraction of AlCl₃ in IL" means the molar amount of aluminum trichloride, divided by the sum of the molar amounts of aluminum trichloride and chloride (IV) used as starting materials. For example, a molar ratio of aluminum chloride to chloride (IV) of 2:1 corresponds to a mole fraction of AlCl₃ of ca. 0.66.

In Entries 5 to 11, "Molar Ratio AlCl₃/PhCOCl" means the ratio of the "excess" of aluminum trichloride, which is the molar amount of aluminum trichloride minus the molar amount of chloride (IV), to the molar amount of benzoyl chloride (II). For example, if 2.25 mmol of AlCl₃ and 1.00 mmol of chloride (IV) are employed per 1.00 mmol of benzoyl chloride used as starting material, the ratio is (2.25 - 1.00) ÷ 1.00 = 1.25.

*T*₁ indicates the temperature at which PhCOCl is added to the AlCl₃/solvent mixture while *T*₂ indicates the reaction temperature during the addition of acetylene.

The conversions, as well as the purities and *E*/*Z* ratios of the isolated products, were determined using gas chromatography (GC). The abbreviation "n.d." means not determined.

The entries in the column headed "Tar" indicate the amount of tar formation (if any) that has been observed.

The isolated yields (Entries 4, 7 and 11) were determined after aqueous/organic extraction, followed by column chromatography (Entry 4 only).

The *E*/*Z* ratios were determined after the isolated material had been stored at 4 °C overnight.

### Example 4

In a similar procedure as described in Example 2, benzoyl chloride (II, R = Ph) was reacted with acetylene in [bmpy]+[Al*ₙ*Cl_{3*n*+1}]⁻, using different molar ratios of aluminum trichloride to benzoyl chloride (as defined above, i.e. the ratio of the "excess" of aluminum trichloride to the molar amount of benzoyl chloride), different mole fractions (as defined above) of aluminum trichloride in the ionic liquid, and different reaction times. The addition of benzoyl chloride to the chloride [bmpy]⁺Cl⁻ was carried out at room temperature while the reaction temperature during the addition of acetylene was 60 °C. The results are compiled in Table 2 below:

**Table 2**

| Entry | Molar Ratio AlCl₃/PhCOCl | Mole Fraction of AlCl₃ in IL | *t*₁ (h) | *t*₂ (h) | Conversion (%) | Isolated Yield (%) |
|---|---|---|---|---|---|---|
| 1 | 0.68 | 0.67 | 0.25 | 2 | 60 | n.d. |
| 2 | 1.3 | 0.67 | 0.25 | 2 | >99 | 74 |
| 3 | 1.3 | 0.67 | 0.25 | 4 | >99 | 73 |
| 4 | 1.3 | 0.67 | 3 | 4 | >99 | 73 |
| 5 | 1.8 | 0.67 | 0.25 | 2 | >99 | 90 |
| 6 | 1.8 | 0.70 | 0.25 | 2 | >99 | 93 |

*t*₁ is the reaction time for the addition of benzoyl chloride to the chloride [bmpy]⁺Cl⁻ while t₂ is the reaction time for the addition of acetylene.

After aqueous/organic extraction, the product purity was in general 97-98 %, based on GC analysis.

### Example 5

The procedure of Example 2 was repeated using various acyl chlorides (II) as starting materials, namely benzoyl chloride (R = phenyl), p-toluoyl chloride (R = *p*-tolyl), *p*-nitrobenzoyl chloride (R = *p*-nitrophenyl), p-bromobenzoyl chloride (R = *p*-bromophenyl), 4-methylpentanoyl (isocaproyl) chloride (R = isopentyl), 3,3-dimethylbutanoyl chloride (R = neopentyl), and acetyl chloride (R = methyl). The medium was [bmpy]⁺[Al*ₙ*Cl_{*n*+1}]⁻ for all experiments. The results are compiled in Table 3 below:

**Table 3**

| **Entry** | **R** | **Yield (%)** | **Selectivity (GC, %)** | ***E*/*Z* Ratio (GC)** |
|---|---|---|---|---|
| 1 | phenyl | 93 | 98 | 90/10 (98/2) |
| 2 | *p*-tolyl | 92 | 97 | 93/7 (98/2) |
| 3 | *p*-nitrophenyl | 72 | >99 | 98/2 |
| 4 | *p*-bromophenyl | 86 | 98 | 98/11 (99/1) |
| 5 | isopentyl | 73 | 97 | 94/6 (98/2) |
| 6 | neopentyl | 98 | 99 | 90/10 |
| 7 | methyl | 75 | 99 | 98/11 |

The reaction conditions were as follows:
Addition of acyl chloride to ionic liquid: 15 min at room temperature for Entries 1-4, 15 min at 0 °C for Entries 5-7.
Addition of acetylene: 2 h at 60 °C for Entries 1-4, 2 h at 0 °C for Entries 5-7

The molar ratio AlCl₃/acyl chloride, as defined above, i.e. the ratio of the "excess" of aluminum trichloride to the molar amount of acyl chloride, was 1.8 while the mole fraction (as defined above) of aluminum trichloride in the respective ionic liquid was 0.70.

The yields in Table 3 are isolated yields, with the exception of Entries 6 and 7 (GC yields). In the preparation of β-chlorovinyl *p*-nitrophenyl ketone (Entry 3) an additional purification step (extraction with hot hexane) was carried out. The *E*/*Z* ratios of the freshly prepared products are given in the last column. Values in parentheses are those observed after the isolated product hat been stored at 4 °C overnight.

## Claims

1. A process for the production of a β-chlorovinyl ketone of formula 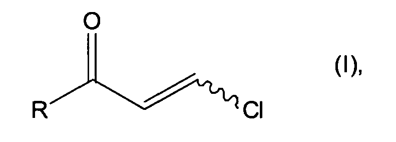 wherein R is selected from the group consisting of linear and branched C₁₋₂₀ alkyl, C₂₋₂₀ alkenyl, C₃₋₁₀ cycloalkyl, optionally substituted aryl, optionally substituted heteroaryl, optionally substituted arylalkyl, optionally substituted arylalkenyl, and optionally substituted heteroarylalkyl, comprising the reaction of an acyl chloride of formula 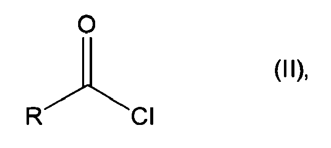 wherein R is as defined above,
with acetylene in a ionic liquid comprising a chloroaluminate of formula
Q⁺ [Al*ₙ*Cl_{3*n*+1}]⁻ (III),
wherein *n* is 1 or 2, and Q⁺ is an organic cation selected from the group consisting of 1,3-dialkylimidazolium, wherein both alkyl groups are independently C₁₋₁₀ alkyl, and optionally alkyl-substituted *N*-(C₁₋₁₀ alkyl)pyridinium.

2. The process of claim 1, wherein Q⁺ is 1-butyl-3-methylimidazolium or *N*-butyl-3-methylpyridinium.

3. The process of claim 1 or 2, wherein R is selected from the group consisting of linear and branched C₁₋₂₀ alkyl and optionally substituted phenyl.

4. The process of any of claims 1 to 3, wherein the chloroaluminate (III) in the ionic liquid has been prepared from the corresponding chloride of formula
Q⁺ Cl⁻ (IV),
wherein Q⁺ is as defined in claim 1 or claim 2, and aluminum trichloride.

5. The process of claim 4, wherein the molar ratio of aluminum trichloride and the chloride (IV) is from 1.5 to 3.0.

6. The process of claim 5, wherein the molar ratio of aluminum trichloride and the chloride (IV) is from 1.8 to 2.5.

7. The process of claim 6, wherein the molar ratio of aluminum trichloride and the chloride (IV) is from 2.0 to 2.3.

8. The process of any of claims 4 to 7, wherein the ratio of the difference of the molar amounts of aluminum trichloride and the chloride (IV) to the molar amount of acyl chloride (II) is from 1.0 to 2.5.

9. The process of claim 8, wherein the ratio of the difference of the molar amounts of aluminum trichloride and the chloride (IV) to the molar amount of acyl chloride (II) is from 1.15 to 2.0.

10. The process of claim 9, wherein the ratio of the difference of the molar amounts of aluminum trichloride and the chloride (IV) to the molar amount of acyl chloride (II) is from 1.3 to 1.8.

11. The process of any of claims 1 to 10, wherein, in a first step, the acyl chloride (II) is added to the ionic liquid comprising the chloroaluminate (III), and then, in a second step, acetylene is added to the resulting mixture.
